# EUROPEAN PATENT SPECIFICATION

(11) **EP 0 721 461 B1**
(45) Date of publication and mention of the grant of the patent: **03.02.1999**
(21) Application number: 94927003.7
(22) Date of filing: 22.09.1994
(51) Int. Cl.: C07J 43/00, C07J 13/00, C07J 41/00

(54) **SYNTHESIS OF 17-(3-PYRIDYL) STEROIDS**
SYNTHESE VON 17-(3-PYRIDYL) STEROIDEN
SYNTHESE DE STEROIDES A BASE DE 17-(3-PYRIDYL)

(30) Priority: 30.09.1993 GB 9320132; 14.07.1994 GB 9414192
(43) Date of publication of application: 17.07.1996
(73) Proprietor: BTG INTERNATIONAL LIMITED, London EC4M 7SB (GB)
(72) Inventor: POTTER, Gerard, Andrew, Widnes, Cheshire WA8 0DP (GB); HARDCASTLE, Ian, Robert, Sutton, Surrey SM1 4RW (GB)
(74) Representative: Percy, Richard Keith
(86) International application number: GB9402054
(87) International publication number: WO9509178

(56) References cited:
- WO-A-93/20097
- HETEROCYCLES, vol.16, no.4, 1981, AMSTERDAM NL pages 521 - 524 J. WICHA ET AL 'Synthesis of17.beta-pyridyl- and 17.beta-pyridonyl-androstane derivatives.'
- JOURNAL OF ORGANIC CHEMISTRY, vol.47, no.26, 17 December 1982, EASTON US pages 5189 - 5191 MIN-JEN SHIAO 'New synthesis of azabufalin from C-17 steroidal ketones.'

## Description

This invention relates to the synthesis of steroids which have a 16, 17-double bond and a 17-(3-pyridyl) substituent.

### Description of the related art

In patent applications GB 2265624A (British Technology Group Limited) and PCT WO 93/20097 (British Technology Group Limited, S.E. Barrie, M. Jarman and G.A. Potter), both filed on 15th March 1993, and published on 6th and 14th October 1993 respectively, we have described 16,17-ene-17-(3-pyridyl) steroids as a class of compounds useful for treatment of androgen- and oestrogen-dependent disorders, especially prostatic and breast cancer respectively. A few of these compounds have previously been mentioned in the literature as intermediates in synthesis, to prepare final products having other uses, but otherwise these compounds were new. Posters have been presented, notably at the SmithKline Beecham Research Symposium, Robinson College Cambridge, England, 25-26 March 1993 and at the British Association for Cancer Research meeting in Sheffield, England, 28-31 March 1993. The Cambridge poster describes the synthesis of an exemplified 16,l7-ene-17-(3-pyridyl) steroid as follows:

Synthesis of this molecule was envisaged via a possible palladium catalysed cross-coupling [G.A. Potter and R. McCague, J. Org Chem. 55, 6184-6187 (1990)] of a steroidal enol triflate (trifluoromethylsulfonate) with a suitable 3-pyridyl nucleophilic coupling partner. This was achieved by using diethyl(3-pyridyl)borane in aqueous THF, with sodium carbonate as nucleophilic activator. The reaction proceeded remarkably efficiently, without possible triflate hydrolysis or ethyl coupling, providing the desired compound in 80% isolated yield (Scheme 1): 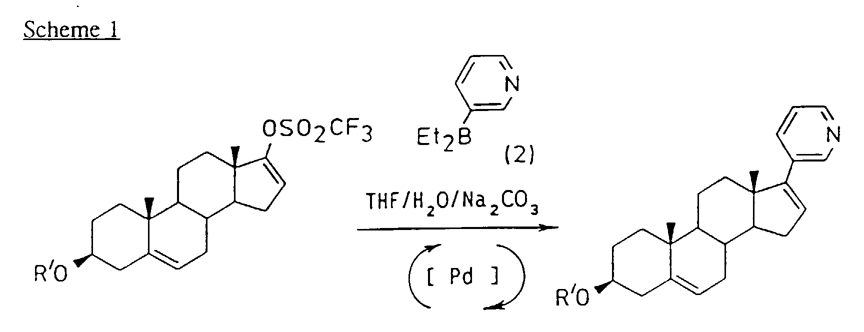 (wherein in the final compound R' = H)

Triflates are expensive starting materials, so an alternative route is desirable. Further, notwithstanding what might appear from a literal reading of the poster, to obtain the 3-ol the reaction has to be carried out on the 3-acetate as protecting group, not the 3-ol. The 3-acetate is then hydrolysed to the 3-ol subsequently in a separate step. The said patent applications propose in outline an alternative route, as follows (Scheme 2): 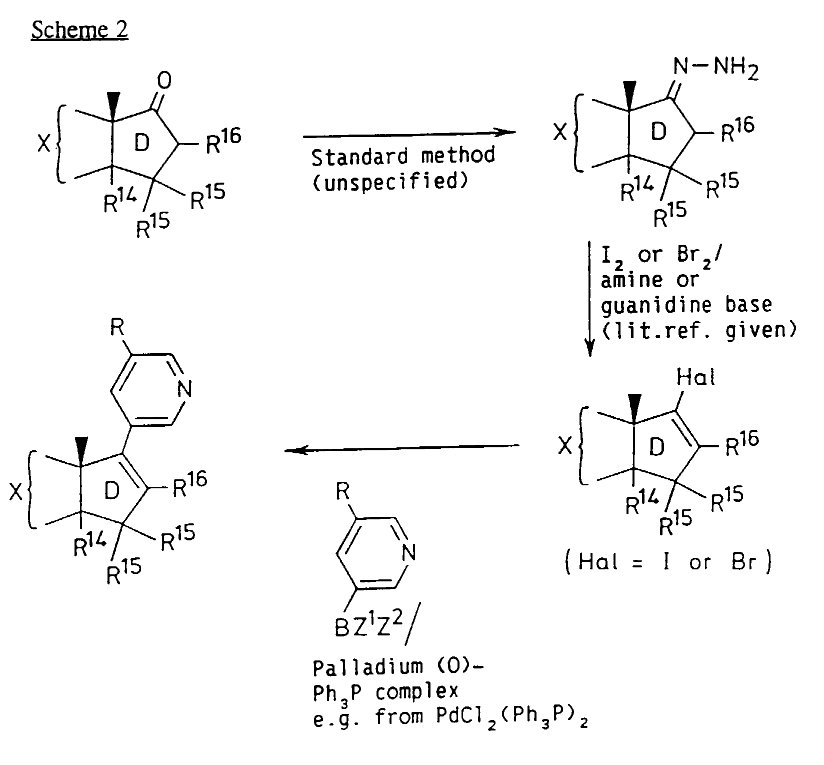 wherein X represents the residue of the A, B and C rings of a steroid, R represents a hydrogen atom or an alkyl group of 1-4 carbon atoms, R¹⁴ represents a hydrogen atom, a halogen atom or an alkyl group of 1 to 4 carbon atoms and each of the R¹⁵ substituents independently represents a hydrogen atom or an alkyl or alkoxy group of 1-4 carbon atoms, a hydroxy group or an alkylcarbonyloxy group of 2 to 5 carbon atoms or together represent an oxo or methylene group or R¹⁴ and one of the R¹⁵ groups together represent a double bond and the other R¹⁵ group represents a hydrogen atom or an alkyl group of 1 to 4 carbon atoms, and R¹⁶ represents a hydrogen atom, halogen atom, or an alkyl group of 1 to 4 carbon atoms, and Z¹ and Z² independently represent hydroxy or alkoxy or alkyl of 1-4 carbon atoms each, preferably ethyl or methoxy.

### Summary of the invention

It has now surprisingly been found that in the preparation of 3β-acyloxy-16, 17-ene-17-(3-pyridyl) steroids, especially the preferred compound, 3β-acetoxy-17-(3-pyridyl)androsta-5,16-diene, of formula (1): 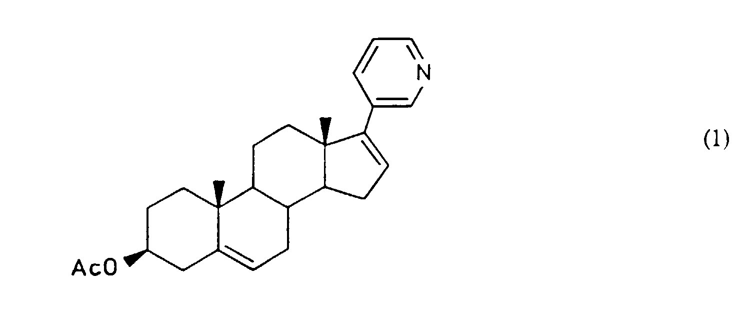 the reaction can be carried out via the vinyl iodide intermediate, but using the unprotected 3β-hydroxy compound. By-products can be reduced either (a) by keeping the proportion of organoboron compound (borane) used in the cross-coupling reaction to not more than 1.2 equivalents per equivalent of steroid or (b) by crystallising the reaction product of the cross-coupling reaction from a mixture of acetonitrile and methanol. This route via the vinyl iodide intermediate is therefore amenable to large scale synthesis.

The principle of the invention may be expressed as a method of preparing a 3β-hydroxy- or 3β-(lower acyloxy)-16,17-ene-17-(3-pyridyl)-substituted steroid, wherein the 3β-(lower acyloxy) group of the steroid has from 2 to 4 carbon atoms, which comprises subjecting a 3p-hydroxy-16,17-ene-17-iodo or -bromo steroid to a palladium complex-catalysed cross-coupling reaction with a (3-pyridyl)-substituted borane, in which the pyridine ring is substituted at the 5-position by an alkyl group of 1 to 4 carbon atoms or is unsubstituted thereat, especially with a said borane of formula (2): 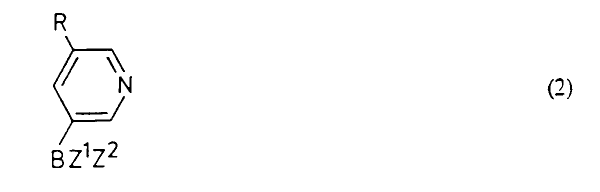 wherein R is a hydrogen atom or an alkyl group of 1-4 carbon atoms and Z¹ and Z² independently represent hydroxy or alkoxy or alkyl or 1-3 carbon atoms each or Z¹ and Z² together represent an alkylenedioxy group of 2 or 3 carbon atoms, in a proportion of at least 1.0 equivalent of boron compound per equivalent of steroid, in an organic liquid. which is a solvent for the 3β-hydroxy steroidal reaction product, and optionally esterifying the 3β-hydroxy reaction product to the 3β-acyloxy ester, which method comprises feature (a) or (b) above.

### Description of the preferred embodiments

Preferably the 17-iodo ("vinyl iodide") or -bromo starting compound has a D-ring with optional substitution in position 14, 15 and/or 16 as shown in Scheme 2. Most preferably it is prepared from the corresponding 17-ketone, conveniently via the corresponding hydrazone. Preferably the vinyl iodide or bromide is unsubstituted in the 14, 15 and 16-positions, in which case it can be prepared from dehydroepiandrosterone (DHEA). In the hydrazination it is preferable to use hydrazine hydrate together with a catalytic amount of a proton provider which is most preferably hydrazine sulfate.

The hydrazone is preferably iodinated with iodine or brominated with bromine in the presence of a strong base such as a tetraalkylguanidine, especially tetramethylguandine which is cheaply and readily available.

In the cross-coupling reaction, the boron compound is preferably a diethylborane or a dimethoxyborane (Z¹ =Z²= Et or OMe). Other boranes include those in which the boron atom is part of a cyclic ether ring e.g. as in Z¹, Z² = 1,2-ethylenedioxy or 1,3-propylenedioxy. In embodiment (a) of this invention the proportion of borane added is at least 1.0, but no more than 1.2 equivalents of boron per equivalent of steroid. preferably about 1.1, but in the embodiment (b) a higher proportion is preferred, of at least 1.2 : 1 to 1.5 : 1 equivalents of boron compound to steroid. The higher proportion will give the better yield of product but also more of the contaminating boron, phosphine and/or palladium compounds. According to embodiment (b), however, these are removed with the acetonitrile solvent. In either embodiment, the palladium compound is a palladium (0) phosphine complex such as tetrakis(triphenylphosphine) palladium (0) or a compound reducible to a palladium (0) phosphine species, especially bis(triphenylphosphine) palladium (II) chloride. The reaction vessel is preferably purged with an inert gas, especially argon or nitrogen, to minimise the possibility of oxidation with a corresponding redox reduction of palladium to the metallic state.

The cross-coupling reaction is preferably carried out in two phases, one aqueous. one organic. The organic phase comprises an organic solvent for the 3β-hydroxy steroidal reaction product, especially tetrahydrofuran (THF). Other cyclic ethers such as dioxane could be used in place of THF. Preferably, a nucleophilic activator, such as sodium carbonate, is used, in which case it is normally present in the aqueous phase.

After the reaction, inorganic salts can be removed by first adding another organic solvent, preferably diethyl ether, which is a solvent for the organoboron contaminants produced in the reaction product, and miscible with the first-mentioned organic solvent (e.g. THF), but immiscible with water, whereafter the organic, e.g. THF-diethyl ether, phase and water (aqueous phase) can be separated. After this separation, various work-up procedures are operable. In one procedure, particularly suited to embodiment (a), the THF and diethyl ether are removed, e.g. evaporated as a mixture, and the remaining reaction product is washed with a third organic solvent, which can be diethyl ether, preferably cooled to below room temperature, most especially to 10°C or lower. The third organic solvent is one in which the 3β-hydroxy steroid reaction product has a low solubility and which, importantly, removes the organoboron compound/s (and also the contaminating phosphine and palladium compound/s). Diethyl ether is preferred.

A different work-up procedure, used in embodiment (b), comprises crystallisation from acetonitrile/methanol. Acetonitrile is a preferred crystallisation solvent to keep boron compound as well as palladium compound in solution and is therefore used in an excess over methanol e.g. an excess of at least 5 : 1 and preferably about 8 : 1 by volume.

To prepare the 3β-acyloxy (alkylcarbonyloxy) compounds. of which the acetoxy compound is preferred, standard acylating (acyl-esterification) agents such as aceryl, propionyl or butyryl chloride or anhydride can be used in step (d). The final product of step (d) may be crystallised direct from hexane, rather than from ethanol/water followed by hexane. Preferably, the work-up procedure comprises reverse phase chromatography, i.e. using a relatively lipophilic solid phase. In this procedure, the chief by-product. a bis-steroidal compound, is preferentially retained on the solid phase and can be eluted with a good separation from the desired product.

The following Examples illustrate the invention. In Example 1, the 3 β-hydroxy product is produced without chromatography, by embodiment (a). In Example 2. the 3β-hydroxy product is not isolated, but in step (d) an impurity has been identified as a 16,17'-bis(steroidal) by-product. This can be removed by reverse phase chromatography, but now that the by-product has been identified, those skilled in the art will be able more easily to identify procedures which will remove it, without the need for chromatography. Further, it is believed that with the higher organoboron : steroid ratios suggested above, the side-reaction leading to this impurity will be reduced.

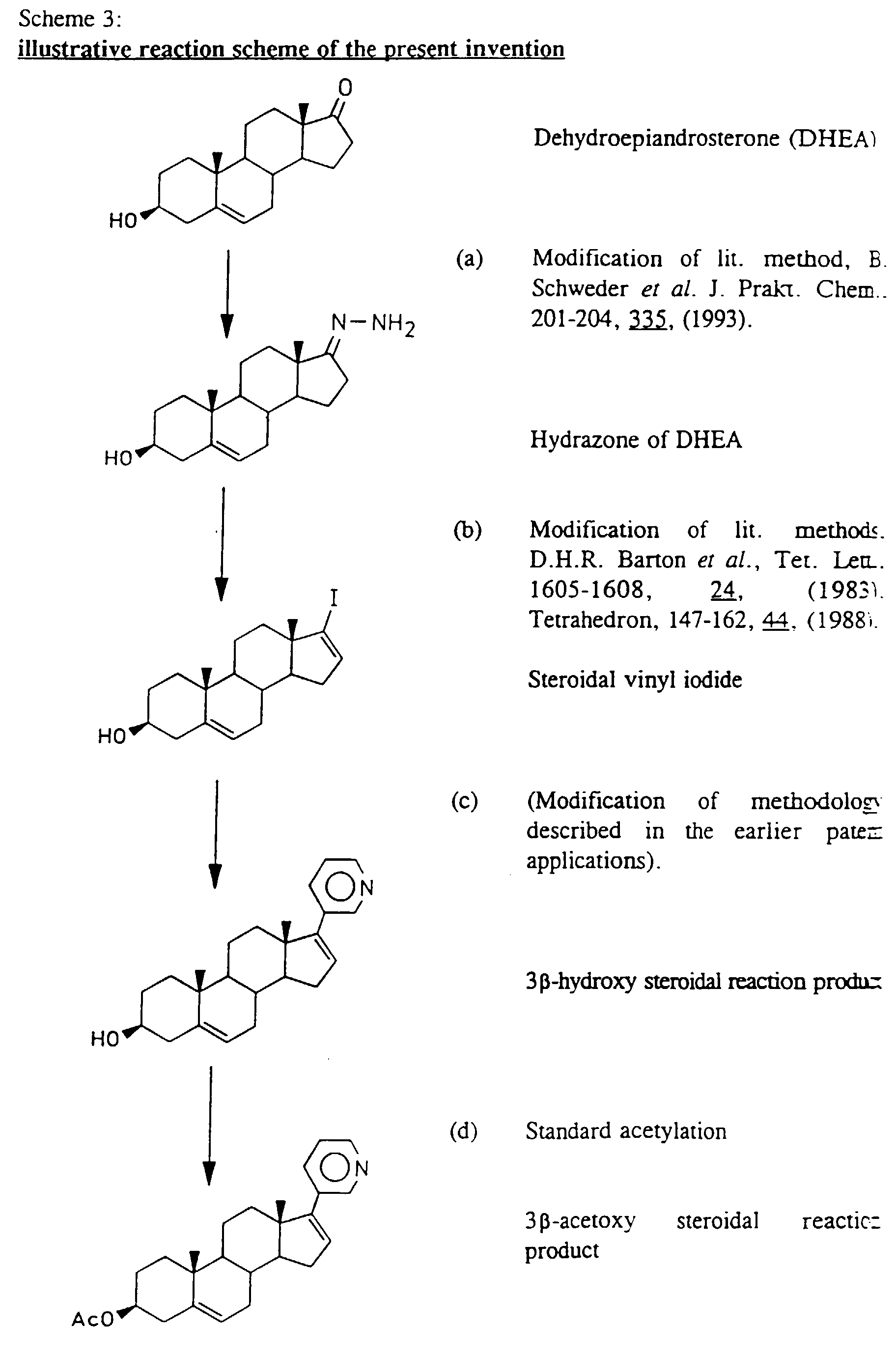

### EXAMPLE 1

This example illustrates embodiment (a).
**(a) Dehydroepiandrosterone-17-hydrazone**
   To a stirred solution of dehydroepiandrosterone (28.8g, 0.1 mol) in ethanol (500ml) was added hydrazine hydrate (19.5ml, 0.4 mol), followed by a solution of hydrazine sulfate (65mg, 0.5 mmol) in water (2ml). After stirring for 3 days the mixture was poured into water (3 litres) to precipitate the product as a white crystalline solid. The product was collected by filtration on a sinter, washed with cold water ( 2 x 50ml), then with Et₂O (50ml). The product was then dried in *vacuo,* firstly over silica gel. and finally over P₂O₅, to give the title compound as a white cyrstalline solid (29.6g, 98%).
   Notes
   1) The method of Schweder *et al.,* p.202, compound No. 2 therein (using triethylamine) gave a very fine crystalline product which was difficult to filter.
   2) The method of Schweder *et al.* p. 203, compound No. 4 therein (using sodium acetate buffer) gave a slightly lower yield (96%) in trial experiments, whereas the modified procedure used above gave a product amenable for filtration, and in excellent yield (98%).
**(b) 17-Iodo-androsta-5,16-dien-3β-ol**
   To a solution of iodine (53.3g, 0.21 mol) in THF (2L), cooled by an ice/water bath to 0°C, was added 1,1,3,3-tetramethylguanidine (63 ml, 57.6g, 0.50 mol).
   A solution of dehydroepiandrosterone-17-hydrazone (30.25g, 0.10 mol) in THF (750ml) was then added slowly to the above iodine solution via a transfer needle over about 2h, whilst maintaining the reaction temperature at 0°C. After all the hydrazone solution was added, the mixture was filtered, and the filtrate concentrated. The remaining oil was then heated on an oil bath for 4h, allowed to cool, and dissolved in Et₂O. The Et₂O solution was washed with 1M HCl until the aqueous phase was acidic, washed with 0.5M NaOH, then 1M Na₂S₂O₃, and finally with water. The Et₂O phase was separated, dried (MgSO₄), and concentrated to give the crude product. Recrystallisation from Et₂O/hexane (3:2) afforded the title compound as off-white crystals (35.8g, 90%).
**(c) 17-(3-Pyridyl)androsta-5,16-dien-3β-ol**
   Diethyl(3-pyridyl)borane (3.23g, 22 mmol) from Aldrich Chemical Co. Ltd. was added to a stirred solution of 17-iodo-androsta-5,16-dien-3β-ol (7.96g, 20 mmol) in THF (120ml) containing bis(triphenylphosphine)palladium (II) chloride (140mg. 0.2 mmol). An aqueous solution of sodium carbonate (2M, 50ml) was then added and the mixture heated, with stirring, by an oil bath at 80°C for 48h, and allowed to cool.
   The mixture was partitioned between Et₂O and water the organic phase was separated, dried (Na₂CO₃) and twice concentrated from Et₂O by evaporation to remove THF (with Et₂O). The residual solid was then washed with Et₂O (100ml), the Et₂O solution decanted off, and the remaining white solid recrystallised from toluene (3.94g, 56%).
   Notes
   1) The time required for completion needs to be made longer than when using the vinyl triflate (48h vs 1h) since it has been found that the vinyl iodide reacts much more slowly.
   2) It has been found that a smaller excess of borane than described in the earlier applications (for the vinyl triflate) aids in isolation of product.
   3) The work-up procedure enables the product to be isolated without chromatography, thereby enabling scaling up.
**(d) 3β-Acetoxy-17-(3-pyridyl)androsta-5,16-diene**
   To a stirred suspension of finely powdered 17-(3-pyridyl)androsta-5,16-dien-3β-ol (3.50g, 10 mmol) in dry diethyl ether (150ml) containing triethylamine (2.3ml, 16 mmol) and dimethylaminopyridine (0.012g, 0.1 mmol) was added acetyl chloride (1.0ml, 14 mmol). The mixture was then stirred at ambient temperature for 12h, over which time a thick white precipitate of triethylammonium chloride had formed. The mixture was then filtered and the filtrate concentrated to afford the crude product which was recrystallised firstly from ethanol/water (1:1), then finally from hexane to afford the title compound (3.30g, 84%).

### EXAMPLE 2

**(a) Dehydroepiandrosterone-17-hydrazone**
   Into a 10L round-bottomed flask, fitted with a magnetic stirrer bar, was placed dehydroepiandrosterone (288g, 1.0 mol) and ethanol (5.0L). To the resultant stirred solution was added hydrazine hydrate (195ml, 4.0 mol), followed by a solution of hydrazine sulfate (0.65g, 0.005 mol) in water (20ml) [note: the hydrazine sulfate dissolved in this volume of water at about 40°C]. After stirring at room temperature for 5 days, water (4.5L) was added, the mixture poured into water (10L), and the white crystalline precipitate allowed to settle. The product was collected by filtration on a sinter, washed with cold water (2 x 500ml), then with Et₂O (2 x 500ml). The product was then dried *in vacuo,* firstly over silica gel, and finally over P₂O₅, to give the title compound as a white crystalline solid, mp 204-206°C (284.8g, 94%).
**(b) 17-Iodo-androsta-5,16 dien-3β-ol**
   A 10L round-bottomed flask, fitted with a magnetic stirrer bar, was charged with iodine (156.1g, 0.615 mol), THF (4.0L; GPR grade), and Et₂O (2.0L; BDH specially dried grade). The resultant stirred solution was cooled by an ice/water bath to 0°C and 1,1,3,3-tetramethylguanidine (188ml, 173g, 1.50 mol) was added. A solution of dehydroepiandrosterone-17-hydrazone from step (a) (90.74g, 0.30 mol) in THF (2.25L) was then added slowly to the above iodine solution via a canula over about 2h, whilst maintaining the reaction temperature at 0°C [note: N₂ is evolved as the hydrazone is added to the iodine solution]. After all the hydrazone solution was added, the mixture was stirred for an additional hour and the precipitate allowed to settle [note: a precipitate of tetramethylguanidium iodide forms during the reaction]. The mixture was then filtered, and the filtrate concentrated to an oil on a rotary evaporator.
   This reaction was carried out a total of three times, thus using in total 272.22g (0.90 mol) of dehydroepiandrosterone-17-hydrazone from step (a). The concentrated residues from the three separate reactions were combined and heated on an oil bath for 4h, then allowed to cool [note: this converts any 17,l7-diiodo by-product into the 17-vinyl iodide product]. This oil was then dissolved in Et₂O (5L), filtered, and further diluted with additional Et₂O (4L).
   The Et₂O solution was washed with aqueous HCl (1M; 3 x 500ml) until the aqueous phase was acidic [note: the ether solution changes colour from brown to yellow when the aqueous phase remains acidified] then finally with water (500ml). The Et₂O phase was separated, dried (MgSO₄), and concentrated to a volume of 3L, then left to allow the product to crystallise. The yellow crystals were collected by filtration on a sinter, washed with hexane (3 x 500ml) and dried under vacuum (335.4g, 94%). Recrystallisation from ethanol-water (5:1) afforded the product as white crystals (297.3g. 83%) mp 175-176°C, lit. mp 173-174°C.
**(c) 17-(3-Pyridyl)androsta-5,16-dien-3β-ol**
   In a 2L round-bottomed flask, fitted with a magnetic stirrer bar, was placed the steroidal 17-iodo product from step (b) (98.0g, 0.246 mol) and this was dissolved in THF (1.1L). The flask was purged with argon and bis(triphenylphosphine)palladium (II) chloride catalyst (1.73g, 0.0025 mol) was added, followed by diethyl(3-pyridyl)borane (43.35g, 0.295 mol). To the resultant orange THF solution was added an aqueous solution of sodium carbonate (2M; 500ml). The flask was fitted with a reflux condenser. and the apparatus purged again with argon. The mixture was then heated under reflux (at about 80°C) with stirring on a stirrer/heating mantle (Electrothermal MA) for 4 days [note: upon completion of the reaction the organic phase darkens in colour from orange to dark orange/brown], then allowed to cool. This reaction was carried out a total of three times, thus using a total of 294.0g (0.74 mol) of the steroidal 17-iodo product from step (b).
   The reaction mixtures were combined and Et₂O (5L) added. The organic phase was separated, washed with water (2L), and left to give a first crop of crystals which were collected by filtration on a sinter. The filtrate was concentrated and the residue redissolved in Et₂O to afford a second crop of crystals. The aqueous phase and washings from the above work-up were extracted with hot toluene (2L) on a steam bath and concentration of the toluene extracts afforded further product. The combined crude product from the above procedures was then dissolved in the minimum volume of hot methanol, filtered through a plug of "Celite" (Registered Trade Mark) and an equal volume of acetonitrile added to the methanol solution. The acetonitrile/methanol solution was then concentrated to half its original volume on a rotary evaporator and the solution left to crystallise. The resultant white crystals were collected by filtration on a sinter. washed with acetonitrile and dried *in vacuo* to constant weight (191.1g, 74%). mp 202-212°C. A second recrystallisation from toluene-methanol (50:1) afforded the product as white crystals (146.8g, 57%) mp 214-218°C, lit. mp 228-229°C.
**(d) 3β-Acetoxy-17-(3-pyridyl)androsta-5,16-diene**
   The following reaction was carried out in a 500ml round-bottomed flask, fitted with a magnetic stirrer bar. To a suspension of the steroidal product from step (c) (26.5g, 0.104 mol) in dry pyridine (200ml), was added acetic anydride (75ml) and the mixture stirred at room temperature for 24h. The pyridine and excess acetic anydride were removed on a rotary evaporator. initially with the water bath at 70°C, and finally at 80°C for 30 min. The resulting oil was dissolved in Et₂O (500ml), washed with saturated aqueous NaHCO₃ (2 x 200ml), dried (Na₂CO₃), and concentrated to an oil which crystallised on standing. ¹H-NMR spectroscopy at this stage showed the product contained about 5% of a 16,17'-bi(steroidal) contaminant, 3β-acetoxy-16-(3'-β-acetoxyandrosta-5',16'-dien-17'-yl)-17-(3-pyridyl)androsta-5,16-diene, which originated as a by-product from the coupling reaction of step (c).
   The product was therefore further purified by preparative flash chromatography using a 9cm diameter column, with silica stationary phase (Merck 15111), eluting with dicholoromethane. The by-product eluted first followed by the desired product. although the separation was incomplete. Fractions containing a significant amount of by-product were combined and subjected to further chromatographic purification.
   The foregoing reaction and purification procedure was carried out a total of four times, thus using a total of 146g (0.418 mol) of the steroidal product from step (c).

The product-containing dichloromethane fractions from the chromatographic purification were concentrated and recrystallised from hexane to afford white crystals which were dried *in vacuo* to constant weight. The total amount of product obtained was 136.0g (83%).

The dichloromethane fractions containing the least by-product were combined, and following recrystallisation from hexane, afforded the title compound as white crystals with mp 142-144°C. Analysis showed this material ("A") contained 6.8% w/w of the bis(steroidal) by-product.

A second crop of white crystals ("B") of the product, containing 21.8% w/w of bis(steroidal) by-product (25g), was obtained.

The two products were purified using reverse phase chromatography. The column was packed with "LiChroprep" (Registered Trade Mark) RP-8 reverse-phase C₈ packing, Art. No. 9324, supplied by E. Merck, Darmstadt, Germany. The course of the chromatography was followed by UV detection at 253 nm, with purity checks by HPLC.

Product "A" (10.17g) was dissolved in 200ml. hot acetonitrile and 40ml. hot methanol, and, after being allowed to cool, the filtrate was applied to a 10cm. diameter column containing about 500g. of the packing. The eluant was 5% 0.05M aqueous ammonium acetate/95% v/v acetonitrile. 7.51g. of product was recovered in fractions 4-10. Fractions (500ml) 4-11 contained the product with some impurities, but not the bis-steroidal by-product. The eluant was changed to 2.5% acetic acid/95.5 % v/v acetonitrile and then to 5% acetic acid/95% v/v acetonitrile. A pink colour seen in fractions 16 and 17 evidenced the bis-steroidal by-product. Fraction 18 was colourless. The column can be washed with 100% acetonitrile, for re-use.

Product "B" (1g) was separated by a similar method except that the product was dissolved initially in 100% acetonitrile and the filtrate applied to a 2cm. column packed with 100g. of the solid phase. Excellent separation of the product was achieved with the aqueous ammonium acetate/acetonitrile eluant.

Although, in this Example, the reverse phase column was used in addition to a conventional column, it is clear that the conventional column achieved little separation of the bis-steroidal by-product and it is intended to omit the conventional column in future preparations.

## Claims

1. A method of preparing a 3β-hydroxy- or 3β- (lower acyloxy) 16,17-ene-17-(β-pyridyl)-substituted steroid, wherein the 3β (lower acyloxy) group of the steroid has from 2 to 4 carbon atoms, which comprises subjecting a 3β-hydroxy-16,17-ene-17-iodo or - bromo steroid to a palladium complex-catalysed cross-coupling reaction with a (3-pyridyl)-substituted borane in which the pyridine ring is substituted at the 5-position by an alkyl group of 1 to 4 carbon atoms or is unsubstituted thereat, in a proportion of at least 1.0 equivalent of borane per equivalent of steroid, in an organic liquid which is a solvent for the 3β-hydroxy steroidal reaction product, and, where the 3β-(lower acyloxy) ester is to be prepared, reacting the resulting 3β-hydroxy steroidal reaction product with an esterifying agent effective to replace the hydroxy group by a said lower acyloxy group, which method comprises (a) carrying out the reaction with not more than 1.2 equivalents of borane per equivalent of steroid or (b) crystallising the product of the cross-coupling reaction from a mixture of acetonitrile and methanol.

2. A method according to claim 1, wherein the 3β-hydroxy steroidal reaction product, with or without isolation, is reacted with an acetyl-esterifying agent to give the corresponding 3β-acetoxy-16,17-ene-17-(3-pyridyl) steroid.

3. A method according to claim 1 or 2, wherein the starting steroid has a D-ring of the following partial formula 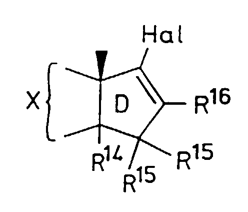 wherein Hal is I or Br, X represents the residue of the A, B and C rings of the steroid. R¹⁴ represents a hydrogen atom, a halogen atom or an alkyl group of 1 to 4 carbon atoms and each of the R¹⁵ substituents independently represents a hydrogen atom or an alkyl or alkoxy group of 1-4 carbon atoms, a hydroxy group or an alkylcarbonyloxy group of 2 to 5 carbon atoms or together represent an oxo or methylene group or R¹⁴ and one of the R¹⁵ groups together represent a double bond and the other R¹⁵ group represents a hydrogen atom or an alkyl group of 1 to 4 carbon atoms, and R¹⁶ represents a hydrogen atom, halogen atom, or an alkyl group of 1 to 4 carbon atoms.

4. A method according to claim 3, wherein the starting steroid is prepared by hydrazinating 3β-hydroxyandrost-5-en-17-one to yield the 17-hydrazone, which is then iodinated or brominated with iodine or bromine, respectively, in the presence of a strong base, to yield the 16,17-ene-17-iodide or -bromide, respectively.

5. A method according to any preceding claim, wherein the (3-pyridyl)-substituted borane is of formula: 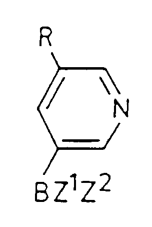 wherein R represents a hydrogen atom or an alkyl group of 1-4 carbon atoms and Z¹ and Z² independently represent hydroxy or alkoxy or alkyl or 1-3 carbon atoms each or Z¹ and Z² together represent an alkylenedioxy group of 2 or 3 carbon atoms.

6. A method according to any preceding claim in which the cross-coupling reaction is carried out in two phases, one of which is aqueous and the other of which comprises the said organic liquid.

7. A method according to any preceding claim wherein feature (b) of Claim 1 is employed and the volumetric ratio of acetonitrile to methanol is at least 5:1.

8. A method according to any preceding claim wherein feature (b) of Claim 1 is employed and the proportion of borane per equivalent of steroid is from 1.2:1 to 1.5:1 .

9. A method according to any preceding claim wherein the ester is prepared and after esterification the product is subjected to reverse phase chromatography.

## Patentansprüche

1. Verfahren zur Herstellung eines 3β-Hydroxy- oder 3β-(Niederacyloxy)-16,17-en-17-(3-pyridyl)-substituierten Steroids, wobei die 3β-(Niederacyloxy)gruppe des Steroids 2 bis 4 Kohlenstoffe enthält, umfassend die Durchführung einer durch einen Palladiumkomplex katalysierten Kreuzkupplungsreaktion zwischen einem 3β-Hydroxy-16,17-en-17-iod- oder -bromsteroid und einem (3-Pyridyl)-substituierten Boran, wobei der Pyridinring in 5-Stellung durch eine Alkylgruppe mit 1 bis 4 Kohlenstoffatomen substituiert oder an dieser Stelle unsubstituiert ist, in einem Anteil von mindestens 1,0 Äquivalent Boran pro Äquivalent Steroid in einer organischen Flüssigkeit, die ein Lösungsmittel für das 3β-Hydroxysteroid-Reaktionsprodukt ist, und wobei der 3β-(Niederacyloxy)ester hergestellt werden soll durch Umsetzung des erhaltenen 3β-Hydroxysteroiden Reaktionsproduktes mit einem Veresterungsmittel, das wirksam ist, die Hydroxygruppe durch die niedere Acyloxygruppe zu ersetzen, wobei das Verfahren umfaßt (a) Durchführung der Reaktion mit nicht mehr als 1,2 Äquivalent Boran pro Äquivalent Steroid oder (b) Auskristallisieren des Produktes der Kreuzkupplungsreaktion aus einem Gemisch aus Acetonitril und Methanol.

2. Verfahren nach Anspruch 1, wobei das 3β-Hydroxysteroid-Reaktionsprodukt, mit oder ohne Isolierung, umgesetzt wird mit einem Acetylveresterungsmittel unter Bildung des entsprechenden 3β-Acetoxy-16,17-en-17-(3-pyridyl)steroids.

3. Verfahren nach Anspruch 1 oder 2, wobei das Ausgangssteroid einen D-Ring der folgenden Teilformel aufweist: 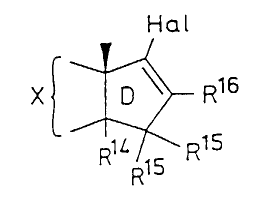 wobei Hal 1 oder Br ist, X den Rest der A-, B- und C-Ringe des Steroids darstellt, R¹⁴ ein Wasserstoffatom, ein Halogenatom oder eine Alkylgruppe mit 1 bis 4 Kohlenstoffatomen ist, und jeder der Substituenten R¹⁵ unabhängig ein Wasserstoffatom oder eine Alkyl- oder Alkoxygruppe mit 1 bis 4 Kohlenstoffatomen, eine Hydroxygruppe oder eine Alkylcarbonyloxygruppe mit 2 bis 5 Kohlenstoffatomen bedeutet, oder (die beiden) zusammen eine Oxo- oder Methylengruppe bedeuten, oder R¹⁴ und eine der Gruppen R¹⁵ zusammen eine Doppelbindung darstellen, und die andere Gruppe R¹⁵ ein Wasserstoffatom oder eine Alkylgruppe mit 1 bis 4 Kohlenstoffatomen bedeutet und R¹⁶ ein Wasserstoffatom, Halogenatom oder eine Alkylgruppe mit 1 bis 4 Kohlenstoffatomen bedeutet.

4. Verfahren nach Anspruch 3, wobei das Ausgangssteroid hergestellt wird durch Behandeln von 3β-Hydroxyandrost-5-en-17-on mit Hydrazin unter Bildung des 17-Hydrazons, das dann mit Iod oder Brom in Gegenwart einer starken Base iodiert bzw. bromiert wird, unter Bildung des 16,17-en-17-Iodids bzw. -Bromids.

5. Verfahren nach einem der vorangehenden Ansprüche, wobei das (3-Pyridyl)-substituierte Boran die Formel hat: 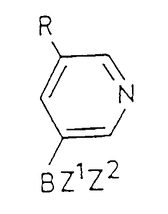 wobei R ein Wasserstoffatom oder eine Alkygruppe mit 1 bis 4 Kohlenstoffatomen bedeutet, und Z¹ und Z² unabhängig Hydroxy oder Alkoxy oder Alkyl mit jeweils 1 bis 3 Kohlenstoffatomen bedeuten, oder Z¹ und Z² zusammen eine Alkylendioxygruppe mit 2 oder 3 Kohlenstoffatomen darstellen.

6. Verfahren nach einem der vorangehenden Ansprüche, wobei die Kreuzkupplungsreaktion in zwei Phasen durchgeführt wird, von denen eine wäßrig ist und die andere die organische Flüssigkeit umfaßt.

7. Verfahren nach einem der vorangehenden Ansprüche, wobei das Merkmal (b) des Anspruchs 1 angewandt wird und das Volumenverhältnis von Acetonitril zu Methanol mindestens 5:1 beträgt.

8. Verfahren nach einem der vorangehenden Ansprüche, wobei das Merkmal (b) des Anspruchs 1 angewandt wird und der Anteil an Boran pro Äquivalent Steroid 1,2:1 bis 1,5:1 beträgt.

9. Verfahren nach einem der vorangehenden Ansprüche, wobei der Ester hergestellt und nach der Veresterung das Produkt der Phasenumkehr-Chromatographie unterworfen wird.

## Revendications

1. Procédé de préparation d'un stéroïde substitué 3β-hydroxy- ou 3β-(acyloxy inférieur)16,17-ène-17- (3-pyridyl), dans lequel le groupe 3β-(acyloxy inférieur) du stéroïde contient 2 à 4 atomes de carbone, qui a pour objet de soumettre un 3β-hydroxy-16,17-ène-17-iodo- ou bromostéroïde à une réaction de couplage croisé catalysée par un complexe au palladium avec un borane (3-pyridyl)-substitué, dans lequel le cycle pyridine est substitué en position 5 par un groupe alkyle ayant 1 à 4 atomes de carbone ou n'est pas substitué, en une proportion d'au moins 1,0 équivalent de borane par équivalent de stéroïde, dans un liquide organique qui est un solvant pour le produit réactionnel 3β-hydroxy-stéroïdique, et lorsque le 3β-(acyloxy inférieur)ester doit être préparé, de faire réagir le produit réactionnel 3β-hydroxy-stéroïdique résultant avec un agent estérifiant efficace pour remplacer le groupe hydroxy par un dit groupe acyloxy inférieur, le procédé comprenant les étapes consistant (a) à effectuer la réaction avec pas plus de 1,2 équivalents de borane par équivalent de stéroïde, ou (b) à faire cristalliser le produit de la réaction de couplage croisé à partir d'un mélange d'acétonitrile et de méthanol.

2. Procédé selon la revendication 1, dans lequel le produit réactionnel 3β-hydroxy-stéroïdique, isolé ou non, est mis à réagir avec un agent d'acétyl-estérification pour donner le 3β-acétoxy-16,17-ène-17-(3-pyridyl)stéroïde.

3. Procédé selon la revendication 1 ou 2, dans lequel le stéroïde de départ présente un cycle D ayant la formule partielle suivante 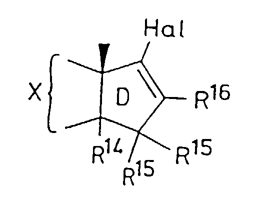 dans laquelle Hal représente I ou Br, X représente le résidu des cycles A, B et C du stéroïde, R¹⁴ représente un atome d'hydrogène, un atome d'halogène ou un groupe alkyle ayant 1 à 14 atomes de carbone et chacun des substituants R¹⁵ représente indépendamment un atome d'hydrogène ou un groupe alkyle ou alcoxy ayant 1 à 4 atomes de carbone, un groupe hydroxy ou un groupe alkylcarbonyloxy ayant 2 à 5 atomes de carbone ou bien représentent ensemble un groupe oxo ou méthylène, ou R¹⁴ et un des groupes R¹⁵ représentent ensemble une double liaison et l'autre groupe R¹⁵ représente un atome d'hydrogène ou un groupe alkyle ayant 1 à 4 atomes de carbone, et R¹⁶ représente un atome d'hydrogène, un atome d'halogène ou un groupe alkyle ayant 1 à 4 atomes de carbone.

4. Procédé selon la revendication 3, dans lequel le stéroïde de départ est préparé par hydrazination de la 3β-hydroxyandrost-5-ène-17-one pour donner la 17-hydrazone, qui est ensuite iodée ou bromée avec de l'iode ou du brome, respectivement, en présence d'une base forte, pour donner le 16,17-ène-17-iodure ou -bromure, respectivement.

5. Procédé selon l'une quelconque des revendications précédentes, dans lequel le borane (3-pyridyl)-substitué répond à la formule : 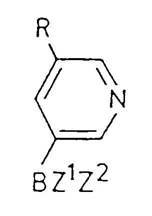 dans laquelle R représente un atome d'hydrogène ou un groupe alkyle ayant 1 à 4 atomes de carbone et Z¹ et Z² représentent indépendamment un groupe hydroxy ou alcoxy ou alkyle ou 1 à 3 atomes de carbone chacun, ou Z¹ et Z² représentent ensemble un groupe alkylènedioxy ayant 2 ou 3 atomes de carbone.

6. Procédé selon l'une quelconque des revendications précédentes, dans lequel la réaction de couplage croisé est réalisée en deux phases, dont l'une est aqueuse et l'autre comprend ledit liquide organique.

7. Procédé selon l'une quelconque des revendications précédentes, dans lequel la caractéristique (b) de la revendication 1 est utilisée et le rapport volumétrique de l'acétonitrile au méthanol est d'au moins 5:1.

8. Procédé selon l'une quelconque des revendications précédentes, dans lequel la caractéristique (b) de la revendication 1 est utilisée et la proportion de borane par équivalent de stéroïde est de 1,2:1 à 1,5:1.

9. Procédé selon l'une quelconque des revendications précédentes, dans lequel l'ester est préparé et après estérification, le produit est soumis à une chromatographie en phase inversée.
